# EUROPÄISCHE PATENTSCHRIFT

(11) **EP 3 487 459 B1**
(45) Veröffentlichungstag und Bekanntmachung des Hinweises auf die Patenterteilung: **04.11.2020**
(21) Anmeldenummer: 17765455.5
(22) Anmeldetag: 14.09.2017
(51) Int. Cl.: A61F 5/41, A61H 19/00

(54) **ERREGUNGSVORRICHTUNG ZUM BESONDERS INTENSIVEN ERREGEN EINES MÄNNLICHEN GESCHLECHTSTEILS**
EXCITATION DEVICE FOR PARTICULARLY INTENSE EXCITATION OF MALE GENITALS
DISPOSITIF D'EXCITATION POUR OBTENIR UNE EXCITATION PARTICULIÈREMENT INTENSE D'UNE PARTIE GÉNITALE MASCULINE

(30) Priorität: 16.09.2016 DE 202016105164 U; 28.02.2017 DE 202017101115 U
(43) Veröffentlichungstag der Anmeldung: 29.05.2019
(73) Patentinhaber: Strobel, Michael, 42929 Wermelskirchen (DE)
(72) Erfinder: Strobel, Michael, 42929 Wermelskirchen (DE)
(74) Vertreter: Lippert Stachow Patentanwälte Rechtsanwälte
(86) Internationale Anmeldenummer: PCT/EP2017/073154
(87) Internationale Veröffentlichungsnummer: WO 2018/050754

(56) Entgegenhaltungen:
- GB-A- 2 450 779
- GB-A- 2 475 901
- US-A- 4 911 176

## Beschreibung

Die Erfindung betrifft eine Erregungsvorrichtung zum besonders intensiven Erregen eines männlichen Geschlechtsteils, vorzugsweise eines Penis, umfassend eine Aufnahmeeinheit, ausgebildet zum Umschließen des erregten männlichen Geschlechtsteils, sowie einen mit dieser verbundenen Halteelement, das mittels einer Schwingungseinheit in Schwingung versetzt werden kann, um die Erregung des Penis zu bewirken.

Derartige Erregungsvorrichtungen werden im Volksmund häufig als "Vibrator" bezeichnet, hier genauer als "Vibrator für einen Mann". Die Aufnahmeeinheit umschließt den erregten Penis und fördert somit einen Rückstau des Blutes im erregten Penis und fördert damit dessen Steifigkeit. Die Aufnahmeeinheit wird dazu genutzt, um die Schwellkörper im Penis mit Blut zu füllen und dieses dann daran zu hindern, wieder zurückzufließen. Damit wird die Erektion stärker und intensiver. Die Aufnahmeeinheit verstärkt somit grundsätzlich die Erektion des Mannes und weitet diese aus. Gleichzeitig wird durch die Schwingungseinheit der Halter in Schwingung gesetzt und auf die Aufnahmeeinheit umfänglich auf den erregten Penis übertragen.

Aus dem Stand der Technik sind verschiedene Vorrichtungen bekannt, die verschieden große Schlaufen bzw. Ringe umfassen, welche zur Erektionsförderung um das männliche Glied und/oder den Hodensack (Skrotum) herum gelegt werden können. Problematisch ist bei bekannten Vorrichtungen, dass auf die jeweilige Größe des Penis im erregten Zustand abgestimmt große Ringe mit entsprechenden Innendurchmessern für die Erzielung einer optimalen Erregung benötigt werden. Vorzugsweise ist der Innendurchmesser der Aufnahmeeinheit, nämlich ca. 3-5 mm, kleiner als der Durchmesser des erregten Penis. Damit werden also eine Vielzahl von verschiedenen Größen benötigt, die aber nie alle Bedürfnisse optimal abdecken können, zumal das Ermittlungsverfahren für den richtigen Innendurchmesser der Aufnahmeeinheit dazu vom individuellen Befinden abhängt. Schließlich bewirkt der Schwinger nicht eine ausreichende Vibration zur Erregung des männlichen Gliedes.

Eine gattungsgemäße Vorrichtung ist bekannt aus GB 2 475 901 A. Vorrichtungen anderer Art sind bekannt aus GB 2 450 7789 A und US 4 911 176 A.

Ausgehend von dem eingangs genannten Stand der Technik liegt der Erfindung die Aufgabe zugrunde, diese Nachteile zumindest teilweise zu vermeiden und insbesondere einen männlichen Vibrator vorzusehen, der variabel einsetzbar ist, also einfach für verschiedene Größen männlicher Penisse verwendbar ist, und mit dem zudem eine besonders intensive Erregung des Penis realisierbar ist.

Die Erfindung betrifft insofern eine Erregungsvorrichtung gemäß Anspruch 1 und die Aufgabe wird in der einfachsten Ausführungsform bereits gelöst durch die kennzeichnenden Merkmale von Anspruch 1.

Die Penisschlaufe kann zur Erregung des Penis entweder um den Penisschaft, die Penisspitze oder den Hodensack gelegt werden.

Die Penisschlaufe ist ein umfänglich geschlossenes Gebilde, mit zwei Enden, die an dem Halteelement zusammenlaufen bzw. an dem Halteelement fixiert sind. Die Penisschlaufe ist annähernd kreis- oder tropfenförmig ausgebildet und kann bei der Befestigung an dem Halteelement eine andere Gestalt annehmen, z.B. spitz zulaufen.

Der besondere Vorteil der erfindungsgemäßen Erregungsvorrichtung liegt darin, dass die Penisschlaufe problemlos relativ groß ausgebildet sein kann, also durchaus einen Außenumfang von 18 bis 25 cm aufweisen kann, so dass diese einfach auf verschiedene Penisgrößen aufgebracht werden kann. Zwischen der Innenseite der Penisschlaufe und dem Penis kann also durchaus ein Freiraum bestehen, weil ein enges Anliegen der Penisschlaufe an dem Penis durch die Drehung des Halteelements und damit bedingte Verkürzung der Penisschlaufe realisierbar ist. Somit liegt die Penisschlaufe immer entsprechend den individuellen Wünschen des Benutzers umfänglich eng an dem Penis.

Als Motor bzw. Antriebseinheit zum Antrieb einer solchen Erregungsvorrichtung kommen insbesondere Oszillationswerkzeuge in Betracht, dabei insbesondere netzunabhängige Systeme mit einem Akku, die unter dem Handelsnamen Multitool-Werkzeug von diversen Herstellern angeboten werden und auf dem Prinzip beruhen, dass eine angetriebene Welle durch einen Exzenter hin- und her bewegt wird. Durch Wechseln einer stirnseitig in dieser Welle sitzenden Befestigungsschraube kann das Halteelement der Erregungsvorrichtung an diesem Oszillationswerkzeug befestigt werden. Ein solcher Oszillations- bzw. Vibrationsschwinger kann mit Schwingungen zwischen 1.000 bis 20.000, vorzugsweise 11.000 bis 20.000 Schwingungen pro Minute auch einstellbar betrieben werden, womit im Gegensatz zu bestehenden Vibratoren eine viel stärkere Erregung des Penis in extrem kurzer Zeit erzielt werden kann. Zweckmäßigerweise ist die Penisschlaufe zumindest an der an das Glied anliegenden Innenseite reibungsverstärkend ausgebildet, weist zumindest dort also zumindest abschnittsweise Gummi, Leder, Latex oder dergleichen auf.

Das Halteelement ist als Schelle ausgebildet, in welche die Penisschlaufe eingeklemmt oder einklemmbar ist, beispielsweise durch Lösen von zwei Befestigungsschrauben, welche zwei zueinander beabstandete Laschen der Schelle gegeneinander drücken.

Die Schelle umfasst ein unteres Befestigungsende zur lösbaren Befestigung an dem Oszillationswerkzeug sowie ein oberes Befestigungsende ausgebildet zur Befestigung der Penisschlaufe.

Wenn die Penisschlaufe direkt an der Schelle befestigt ist, weist die Schelle vorzugsweise zwei zueinander beabstandete Laschen auf, zwischen denen die freien Enden der Penisschlaufe einklemmbar sind, z.B. mittels Nieten oder Schrauben, welche die freien Enden der Penisschlaufe fixieren und die Laschen zusammendrücken. Als besonders erregend hat sich eine elastische Penisschlaufe erwiesen, weil sich diese besonders gut vollumfänglich umschließend an die Außenseite des Penis anlegt. Eine besonders einfache Lösung stellt die Ausbildung der Penisschlaufe als Fensterbankabdichtung dar, welche eine Breite von ca. 25 bis 50 mm aufweist, vorzugsweise eine Breite von ca. 30 mm und eine Dicke von ca. 30 mm aufweist.

Bei einer Weiterentwicklung kann die Penisschlaufe größenverstellbar ausgebildet sein, z.B. mittels einer niederdrückbaren Klemme, womit sich der Innendurchmesser der Penisschlaufe anpassen lässt.

Eine bevorzugte Weiterentwicklung sieht einen Zwischenhalter zwischen dem Halteelement (Schelle) zur Befestigung an der Antriebseinheit und der Penisschlaufe vor. Dieser Zwischenhalter ist vorzugsweise ausgebildet zur lösbaren Befestigung an dem Halteelement, insbesondere kann dieser einen ersten Einschub zum Einstecken des Halteelements aufweisen und umfasst ferner Verbindungsmittel zur Befestigung an dem Halteelement; besonders bevorzugt Schrauben.

Die Penisschlaufe ist vorzugsweise lösbar an dem Halteelement und/oder dem Zwischenhalter fixierbar ausgebildet.

Insbesondere zur Beabstandung des Zwischenhalters von dem Oszillationswerkzeug hat es sich als zweckmäßig erwiesen, dass die Schelle zwischen dem unteren und dem oberen Befestigungssende in Längserstreckungsrichtung geköpft ist, vorzugsweise in einem Winkel von ca. 45 Grad.

Bevorzugt weist der Zwischenhalter zwei miteinander in Einbaulage verbindbare Elemente, z.B. eine Halbschale und einen Einsatz auf, welche in Einbaulage zwischen sich die freien Enden der elastischen Penisschlaufe aufnehmen. Bei einer bevorzugten Ausführungsform ist zumindest ein erstes Schlaufenende der Penisschlaufe lagefixiert, z.B. verschraubt, und ein zweites Schlaufenende lösbar einstellbar, aber definitiv fixiert, z.B. über entlang der Längsrichtung des zweiten Schlaufenendes ausgebildeten Löchern, die auf entsprechende Vorsprünge an der Außenseite des Halters befestigbar sind.

Um ein unbeabsichtigtes Lösen zu vermeiden, was für das Lustempfinden sehr störend sein könnte, umfasst der Zwischenhalter vorzugsweise einen Einklemm- oder Umlenkbereich für mindestens einen Schenkel der Penisschlaufe. Dieser kann z.B. ausgebildet sein als vorstehender Steg, der besonders bevorzugt einstückig an dem Halteelement angeformt ist, aber auch als getrenntes Bauteil ausgebildet sein kann.

Der Zwischenhalter umfasst bevorzugt eine schalenförmige Aufnahme, also ein plattenartiges Element mit einem umlaufenden Rand zur Bildung eines Aufnahmeraums, an dem sowohl das Halteelement fixierbar ist als auch die beiden Enden der Penisschlaufe befestigbar sind, und zwar bevorzugt längenverstellbar. Die bevorzugte Ausführungsform des Zwischenhalters umfasst neben der Aufnahme ferner eine Andrückplatte, welche in die Aufnahme einsetzbar ist, um die Penisschlaufe in der vorgegebenen Solllänge zu fixieren.

Bevorzugt drückt diese Andrückplatte mit der Unterseite auf das erste Befestigungsende der Penisschlaufe, die Penisschlaufe durchläuft dann eine Umlenkung an der Haltereinheit, läuft zurück und wird an der Außenseite an der Andrückplatte befestigt, bevorzugt mittels Vorsprüngen, Noppen oder dergleichen, welche in Löcher an der Penisschlaufe eingreifen, um diese an verschiedene Penisgrößen anzupassen.

Durch diese Ausgestaltung können innerhalb der Penissschlaufe auch verschiedene Einsätze lösbar eingebracht werden, z.B. mit verschiedenen stimulierenden Oberflächenstrukturen, wie Noppen, Rillen und dergleichen oder auch ein Einsatz, der ein Gleitmittel zur Vermeidung von Verletzungen abgibt.

Die Penisschlaufe ausgebildet und bestimmt zur Verwendung mit einer Erregungsvorrichtung der beschriebenen Art.

Diese Penisschlaufe ist also erfindungsgemäß ausgebildet zur direkten Befestigung mit einem als Schelle ausgebildeten Halteelement, welches seinerseits mit einem Oszillations- bzw. Schwingungswerkzeug verbindbar ist, oder einem an dieser Schelle befestigten Zwischenhalter.

Die Penisschlaufe ist ausgebildet als ein im Wesentlichen streifenförmiger, also rechteckig länglicher Grundkörper mit zwei sich im Wesentlichen parallel zueinander erstreckenden Längsseiten, die an den beiden Stirnenden als Befestigungsenden ausgebildet sind.

Der Grundkörper weist eine Schlaufenaußen- und eine Schlaufeninnenseite auf. Die Schlaufeninnenseite ist ausgebildet zur Anlage an und zum Umschließen des Penis.

Bevorzugt besteht die streifenförmige Penisschlaufe aus einem elastischen Material, wobei sich insbesondere EPDM und/oder Silikon als besonders zweckmäßig erwiesen hat.

Zur Bildung der Penisschlaufe werden die Befestigungsenden in einer Einbaulage zur Bildung eines geschlossenen Schlaufenkörpers zusammengelegt und in dieser Sollposition miteinander verbunden zur Anlage und zum Umschließen des Penis.

Diese Befestigungsenden sind zur Bildung eines umfänglich geschlossenen Schlaufenkörpers gegen- bzw. übereinander legbar und in dieser Sollposition fixierbar, entweder an der Schelle, an einem Zwischenhalter oder einem anderen Fügepartner. Vorzugsweise umfassen die Befestigungsenden hierfür eine Öffnung, einen Stift oder dergleichen, mit denen Sie zur Fixierung in Sollposition verbindbar sind.

Bevorzugt beträgt das Verhältnis der Länge der Längsseiten zu der Länge der Stirnseiten zwischen 5 bis 7:1, besonders bevorzugt etwa 6:1.

Die so durch Zusammenlegen der Befestigungsenden gebildete Penisschlaufe umfasst somit eine in Einbaulage dem Penis zugewandte Schlaufeninnenseite sowie eine von dem Penis abgewandte Schlaufenaußenseite.

An der Schlaufenaußenseite sind Befestigungsmittel zur Befestigung an einem Zwischenhalter oder direkt mit dem Halter bzw. der Schelle vorgesehen. Insbesondere können diese Befestigungsmittel in definierten Abständen entlang der Längsachse vorgesehene Befestigungsnoppen, Befestigungsvorsprünge oder dergleichen mit einem oberseitigen Rastrand zum lösbaren Einrasten mit dem jeweiligen Fügepartner umfassen. Auf diese Weise ist die Penisschlaufe vorzugsweise werkzeuglos besonders einfach auf die benutzerindividuelle und für das jeweilige Lustempfinden geeignete Länge einstellbar.

Ausführungsformen sehen vor, dass die Befestigungsnoppen bzw. Befestigungsvorsprünge in gleichmäßigen Abständen (äquidistant) mit der Schlaufenaußenseite ausgebildet sind, also von der äußeren Mantelfläche Schlaufenaußenseite radial nach außen abstehen.

Eine andere Ausführungsform sieht vor, dass die Befestigungsvorsprünge bzw. -noppen in zwei Reihen nebeneinander laufend auf der Schlaufenaußenseite ausgebildet sind, wobei für eine besonders feine Einstellung auch ein Versatz der Reihen der Befestigungsvorsprünge bzw. -noppen zueinander möglich ist.

Die dem Penis in Einbaulage zugewandte Schlaufeninnenseite kann bevorzugt Mittel zur Erhöhung des Anpressdruckes auf das Penisglied umfassen, wie z.B. mindestens eine sich in Längsrichtung auf der Schlaufeninnenseite erstreckende Rippe sowie mehrere Reihen, die sich auch versetzt voneinander in Längsrichtung erstrecken können.

Als besonders zweckmäßig hat sich erwiesen, dass die Materialstärke der Penisschlaufe so groß ist wie die Materialstärke der mindestens einen Rippe bzw. der Noppen. Besonders bevorzugt betragen beide Werte etwa 3 mm.

Die Penisschlaufe und die Vorsprünge können aus einem einheitlichen elastischen Material bestehen, z.B. einem EPDM mit einer SHORE-Härte von ca. 60 +/- 5. Eine Weiterentwicklung sieht vor, dass die Schlaufeninnenseite zumindest teilweise aus einem weicheren Material bzw. einem Material mit einem höheren Reibungskoeffizienten besteht, z.B. Silikon mit einer SHORE-Härte von ca. A25. Ein ebenfalls gut im Humanbereich einsetzbares Material ist Latex, welches ebenfalls gute Reibeigenschaften aufweist.

Allgemein ist die Verwendung von Materialien vorteilhaft, welche gut in dem Humanbereich einsetzbar sind, also z.B. auch Latex, welches elastisch ist, einen guten Reibungskoeffizienten aufweist und gut zu reinigen ist. Andere Materialien sind in diesem Rahmen einsetzbar.

In der folgenden ausführlichen Beschreibung wird auf die beigefügten Zeichnungen Bezug genommen, die Teil dieser Erfindungsbeschreibung bilden und in denen zur Veranschaulichung spezifische Ausführungsformen gezeigt sind, mit denen die Erfindung ausgeübt werden kann. In dieser Hinsicht wird Richtungsterminologie wie etwa "oben", "unten", "vorne", "hinten", "vorderes", "hinteres", usw. in Bezug auf die Orientierungen der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Die folgende ausführliche Beschreibung ist nicht im einschränkenden Sinne aufzufassen. Im Rahmen dieser Beschreibung werden die Begriffe "verbunden", "angeschlossen" sowie "integriert" verwendet zum Beschreiben sowohl einer direkten als auch einer indirekten Verbindung, eines direkten oder indirekten Anschlusses sowie einer direkten oder indirekten Integration. In den Figuren werden identische oder ähnliche Elemente mit identischem Bezugszeichen versehen, soweit dieses zweckmäßig ist. Bezugszeichenlinien sind Linien, die das Bezugszeichen mit dem betreffenden Teil verbinden. Ein Pfeil hingegen, der kein Teil berührt, bezieht sich auf eine Einheit oder Baugruppe, auf die er gerichtet ist. Die Figuren sind im Übrigen nicht unbedingt maßstäblich. Zur Veranschaulichung von Details können möglichweise bestimmte Bereiche übertrieben groß dargestellt sein. Darüber hinaus können die Zeichnungen plakativ vereinfacht sein und enthalten nicht jedes bei der praktischen Ausführung gegebenenfalls vorhandene Detail. Die Begriffe "oben" und "unten" beziehen sich auf die Darstellung in den Figuren.

Es zeigen:

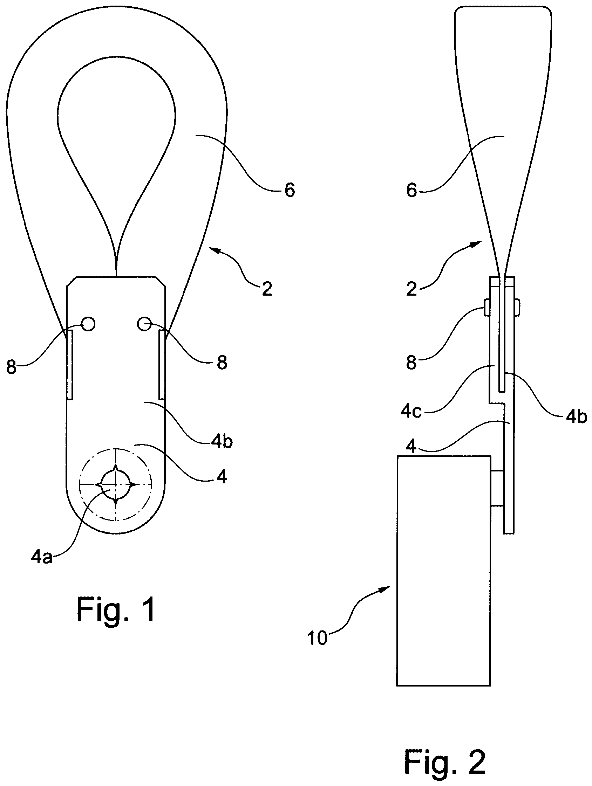

- Figur 1: eine Frontansicht einer erfindungsgemäßen Erregungsvorrichtung; und
- Figur 2: eine Seitenansicht der Erregungsvorrichtung mit einer angedeuteten Antriebseinheit;
- Figur 3: eine perspektivische Ansicht einer zweiten Ausführungsform der erfindungsgemäßen Erregungsvorrichtung mit einem Zwischenhalter;
- Figur 4: eine Seitenansicht der Erregungsvorrichtung;
- Figuren 5A-5C: eine Frontansicht, eine Seitenansicht und eine Draufsicht der Andrückplatte des Zwischenhalters;
- Figur 6A- 6C: eine Frontansicht, eine Seitenansicht und eine Draufsicht einer Aufnahme des Zwischenhalters eines Aufnahmeelements;
- Figur 7: eine isometrische Ansicht einer ersten Ausführungsform einer erfindungsgemäßen Penisschlaufe; und
- Figur 8: eine isometrische Ansicht einer zweiten Ausführungsform einer erfindungsgemäßen Penisschlaufe.

Demnach besteht die in ihrer Gesamtheit mit 2 gekennzeichnete Erregungsvorrichtung aus einem als Schelle 4 ausgebildeten Halteelement und einer in diesem angeordneten Penisschlaufe 6.

Die vorliegend als Metallstanzteil ausgebildete Schelle 4 umfasst einen unteren Abschnitt mit einer Durchgangsöffnung 4a zum Durchstecken einer Befestigungsschraube oder Mutter eines nur schematisch angedeuteten Oszillationswerkzeugs 10. Am oberen Ende mit der Schelle 4 sind zwei voneinander beabstandete Laschen 4b und 4c ausgebildet, die über die Schrauben 8 oder Nieten miteinander verbunden werden können und zwischen denen die freien Enden der Penisschlaufe 6 fixiert sind. Die Penisschlaufe 6 ist also mit den freien Enden flach in die Laschen 4b, 4c der Schelle 4 befestigt und dreht sich dann jeweils um 90° aus der durch die Laschen 4b, 4c der Schelle 4 definierten Ebene heraus bis zum oberen Scheitelpunkt und geht dann wieder unter Drehung von 90° zurück zwischen die Laschen 4b, 4c der Schelle 4.

Die Penisschlaufe 6 ist vorliegend als einteiliges Kautschukband mit einer Breite von ca. 2,5 cm und einer Länge von ca. 20 cm ausgebildet.

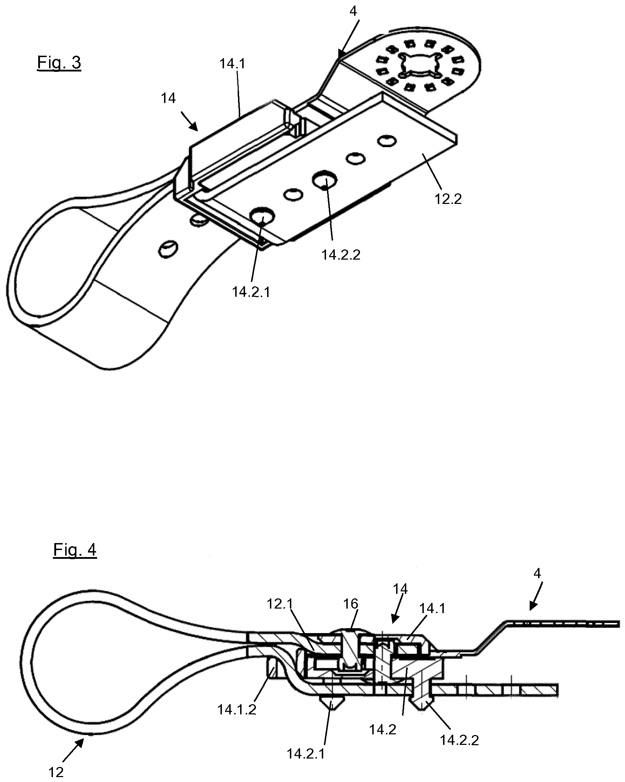

Die Penisschlaufe kann nun um den Penis oder den Hodensack gelegt werden und durch das Oszillationswerkzeug angetrieben werden, womit der Penis enorm erregt wird. Vorzugsweise wird dabei eine Schutzlage zwischen der Innenseite der Penisschlaufe und dem Penis eingebracht, um eine Verletzung durch zu starke Reibung zu verhindern. Bei der in den Figuren 3 und 4 dargestellten zweiten Ausführungsform ist ein Zwischenhalter 14 zwischen der Pennisschlaufe 12 und der Schelle 4 angeordnet, welche eine einfache Verstellung der Länge der Penisschlaufe 12 ermöglicht.

Dieser Zwischenhalter 14 umfasst eine schalenförmige Aufnahme 14.1 sowie eine in diesen einsetzbare Andrückplatte 14.2.

Die Penisschlaufe 12 ist mit ihrem in der Figur oberen, ersten Schlaufenende 12.1 durch eine rückseitige Öffnung 14.1.1. in die schalenförmige Aufnahme 14.1 des Zwischenhalters 14 eingeschoben und am Boden dieser schalenförmigen Aufnahme 14.1 mittels zwei Schrauben 16 fixiert, welche gleichzeitig die Schelle 4 an der Aufnahme 14.1 und somit an dem Zwischenhalter 14 befestigt. Sodann ist die Penisschlaufe 12 so zur Schlaufe umgelegt und das zweite Schlaufenende 12.2 ist umgelenkt zurückgeführt durch einen vorderseitigen Umlenkbereich umfassend einen vom Vorderende der Aufnahme 14.1 beabstandeten Umlenksteg 14.1.2. und liegt schließlich auf der außenseitigen Andrückplatte 14.2 des Zwischenalters 14 auf, die ebenfalls über die Schrauben 16 mit der Aufnahme 14.1 verbunden ist und außenseitig mit zwei abragenden Vorsprüngen 14.2.1, 14.2.2 versehen ist, die in Längsrichtung angeordnet sind und in Einbaulage in zwei Öffnungen bzw. Löcher des zweiten Endes 12.2 der Penisschlaufe 12 eingreifen, um diese längenmäßig in der Solllänge zu fixieren.

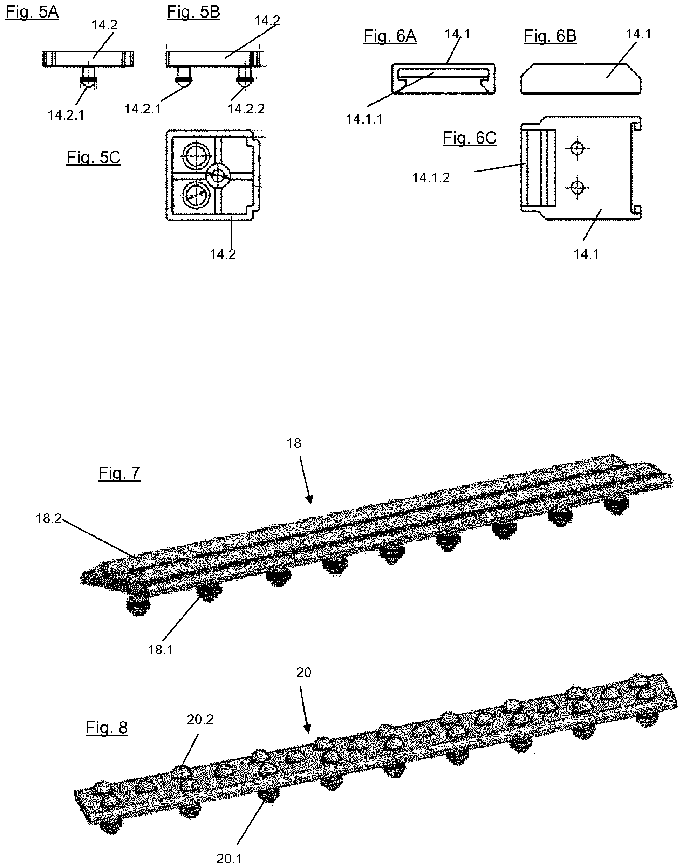

Die Figuren 7 und 8 zeigen jeweils perspektivische Ansichten von erfindungsgemäßen Penisschlaufen 18, 20. Jede dieser Penisschlaufen weist eine Länge von ca. 186 mm und eine Breite von ca. 30 mm auf.

Jede Penisschlaufe 18, 20 besteht aus einem im Wesentlichen streifenförmigem und rechteckigen elastischen Körper, der eine etwa 6-fach so lange Länge wie Breite aufweist und auf der in den Figuren oberen Seite eine Schlaufeninnenseite sowie auf den in den Figuren unteren Seite eine Schlaufenaußenseite bildet.

Mittig auf der Schlaufenaußenseite sind bei beiden Ausführungsformen äquidistant zueinander beabstandet mehrere Befestigungsnoppen 18.1; 20.1 angeformt, die am oberen Ende je eine pilzförmige Verbreiterung aufweisen, mit welchem diese Befestigungsnoppen elastisch verrastend mit entsprechenden Öffnungen des Zwischenhalters, dem Halter oder einem anderen Fügepartner lösbar verbindbar sind.

An der jeweiligen Schlaufeninnenseite sind bei der Ausführungsform gemäß Figur 7 parallel zueinander entlang der Längsachse voneinander beabstandete und von der Oberfläche der Schlaufeninnenseite abragende Lustrippen 18.2 ausgebildet oder gemäß der Ausführungsform gemäß Figur 8 in unterschiedlichen Abständen zueinander halbkugelförmige Lustnoppen 20.2 vorgesehen, von denen jeweils zur Vereinfachung nur eins mit einem Bezugszeichen versehen ist.

Die Lustrippen 18.2 oder die Lustnoppen 20.2 dienen zur Steigerung des Lustgefühls und können angepasst an den jeweiligen Anwendungsfall und das zu erzielende Lustempfinden ausgebildet sein. Somit lassen sich durch die erfindungsgemäße Ausgestaltung des Halters und der Penisschlaufen einfach verschiedene Lustszenarien für den Anwender umsetzen, also unterschiedliche Penisschlaufen in den Zwischenhalter 14 einsetzen. Der Gegenstand der vorliegenden Erfindung ergibt sich nicht nur aus dem Gegenstand der einzelnen Patentansprüche, sondern aus der Kombination der einzelnen Patentansprüche untereinander. Alle in den Unterlagen - einschließlich der Zusammenfassung - offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

### Bezugszeichenliste

- 2: Erregungsvorrichtung
- 4: Schelle
- 4a: Durchgangsöffnung
- 4b, 4c: Lasche
- 6: Penisschlaufe
- 8: Schraube
- 10: Oszillationswerkzeug
- 12: Penisschlaufe
- 12.1: erstes Schlaufenende
- 12.2: zweites Schlaufenende
- 14: Zwischenhalter
- 14.1: Aufnahme
- 14.1.1.: Öffnung
- 14.1.2.: Umlenksteg
- 14.2: Andrückplatte
- 14.2.1.: Vorsprung
- 14.2.2.: Vorsprung
- 16: Schraube
- 18: Penisschlaufe
- 18.1: Befestigungsnoppen
- 18.2: Lustrippe
- 20: Penisschlaufe
- 20.1: Befestigungsnoppen
- 20.2: Lustnoppen

## Patentansprüche

1. Erregungsvorrichtung (2) zum intensiven Erregen eines männlichen Geschlechtsteils, vorzugsweise eines Penis, umfassend eine Aufnahmeeinheit ausgebildet zum Umschließen eines männlichen Geschlechtsteils sowie einem mit dieser verbundenen Halteelement, das mittels eines Antriebs, Schwingers oder dergleichen n Schwingung versetzbar ist, wobei die Aufnahmeeinheit als elastische, umfänglich geschlossene Penisschlaufe (6; 12; 18) mit zwei Schlaufenenden ausgebildet ist, die an dem Halteelement zusammenlaufen bzw. an dem Halteelement fixiert sind, wobei das Halteelement zur Befestigung an einem Oszillationswerkzeug(10) ausgebildet ist, und wobei das Halteelement als Schelle (4) ausgebildet ist, die ein unteres Befestigungsende mit einer Durchstecköffnung (4a) zur lösbaren Befestigung an dem Oszillationswerkzeug (10) sowie ein oberes Befestigungsende zur Befestigung mit den Schlaufenenden der Penisschlaufe (6) umfasst.

2. Erregungsvorrichtung (2) nach Anspruch 1, wobei die Schelle (4) zwei beabstandete Laschen (4b, 4c) aufweist, zwischen denen die Befestigungsenden freien Enden der Penisschlaufe einklemmbar sind.

3. Erregungsvorrichtung (2) nach Anspruch 1 oder 2, wobei die Schelle (4) zwischen einem unteren und dem oberen Befestigungssende in Längserstreckungsrichtung geköpft ist, vorzugsweise in einem Winkel von ca. 45 Grad.

4. Erregungsvorrichtung (2) nach einem der vorhergehenden Ansprüche, wobei die Penisschlaufe (6) zumindest an einer Innenseite ausgebildet ist zur Erzeugung von Reibung.

5. Erregungsvorrichtung (2) nach einem der vorhergehenden Ansprüche, wobei die Penisschlaufe (6) größenverstellbar ausgebildet ist.

6. Erregungsvorrichtung (2) nach den vorhergehenden Ansprüchen, wobei diese einen Zwischenhalter (14) ausgebildet zur Verbindung mit dem Halteelement und der Pennisschlaufe (12) umfasst.

7. Erregungsvorrichtung (2) nach Anspruch 6, wobei der Zwischenhalter (14) ausgebildet ist zur werkzeuglosen Verstellung des Umfangs der Pennisschlaufe (12).

8. Erregungsvorrichtung (2) nach Anspruch 6 oder 7, wobei der Zwischenhalter einen Aufnahmeraum für den Einsatz des Halteelements und mindestens eines ersten Schlaufenende (12.1) der Penisschlaufe (12) bildet.

9. Erregungsvorrichtung nach Anspruch 6 bis 8, wobei der Zwischenhalter (14) Vorsprünge umfasst, welche in verschiedene Löcher eines zweiten Schlaufenendes (12.2) der Pennisschlaufe (12) einsetzbar und in dieser Fixierung für die Solllänge fixierbar sind.

10. Erregungsvorrichtung (2) nach Anspruch 6 bis 9, wobei der Zwischenhalter (14) eine schalenförmige Aufnahme (14.1) mit einem umlaufenden Rand zur Bildung eines Aufnahmeraums aufweist, an dem sowohl die Schelle (4) als auch die beiden Schlaufenenden der Penisschlaufe (6) fixierbar sind, und dass der Zwischenhalter (14) eine Andrückplatte (14.2) umfasst, welche in die Aufnahme (14.1) einsetzbar ist, um die Penisschlaufe (6) in der vorgegebenen Solllänge zu fixieren.

11. Erregungsvorrichtung (2) nach Anspruch 10, wobei die Andrückplatte (14.2) mit einer Unterseite auf das erste Befestigungsende der Penisschlaufe (6) drückt, die Penisschlaufe (6) dann eine Umlenkung an der Halteeinheit durchläuft und dann zurückläuft und an der Außenseite an der Andrückplatte (14.2) befestigbar ist.

12. Erregungsvorrichtung (2) nach Anspruch 11, wobei an der Außenseite der Andrückplatte (14) Vorsprünge (14.2.1, 14.2.2) vorgesehen sind, welche in Löcher an der Penisschlaufe (6) eingreifen, um diese an verschiedene Penisgrößen anzupassen.

13. Erregungsvorrichtung (2) nach einem der vorhergehenden Ansprüche, wobei diese einen Umlenkbereich für die Penisschlaufe (12) umfasst.

14. Erregungsvorrichtung (2) nach einem der vorhergehenden Ansprüche, wobei an der Schlaufenaußenseite Befestigungsmittel zur Befestigung an einem Zwischenhalter oder mit der Schelle (4) vorgesehen sind und wobei an der Schlaufeninnenseite mindestens eine Erhöhung zur Erhöhung des Anpressdruckes auf den Penis aufweist.

15. Erregungsvorrichtung (2) nach Anspruch 1 bis 14, wobei die Erhöhung als mindestens eine sich einer Schlaufenlängsrichtung erstreckende Lustrippe (18.2) und/oder ein Lustnoppen (20.2) ausgebildet ist.

## Claims

1. Excitation device (2) for the intense excitation of male genitals, preferably a penis, including a receiving unit, configured to enclose male genitals, as well as a holding element connected with said receiving unit, which holding element, by means of a drive, vibrating element, or similar, can be made to vibrate, **CHARACTERIZED IN THAT** the receiving unit is configured as an elastic, circumferentially closed a penis loop (6; 12, 18) having two loop ends that converge on the holding element and are fixed on the holding element, respectively, **in that** the holding element is configured for fastening to an oscillation tool (10), and **in that** the holding element is configured as a clamp (4) comprising a lower fastening end with a through-opening (4a) for detachably fasting to the oscillation tool (10) as well as an upper fastening end for fasting with the loop ends of the penis loop (6).

2. Excitation device (2) according to claim 1, **CHARACTERIZED IN THAT** the clam (4) comprises two spaced tabs (4b, 4c) in between which the free ends of the penis loop are clampable.

3. Excitation device (2) according to claim 1 or 2, **CHARACTERIZED IN THAT** the clamp (4) is bent along the direction of longitudinal extension between a lower und an upper fastening end, preferably at an angle of ca. 45 degrees.

4. Excitation device (2) according to one of the preceding claims, **CHARACTERIZED IN THAT** the penis loop (6) is configured, at least on an inner side, for the generation of friction.

5. Excitation device (2) according to one of the preceding claims, **CHARACTERIZED IN THAT** the penis loop (6) is configured size-adjustably.

6. Excitation device (2) according to one of the preceding claims, **CHARACTERIZED IN THAT** this device includes an intermediate bracket (14) configured for the connection with the holding element and the penis loop (12).

7. Excitation device (2) according to claim 6, **CHARACTERIZED IN THAT** the intermediate bracket (14) is configured for the adjustment, free of tools, of the circumference of the penis loop (12).

8. Excitation device (2) according to claim 6 or 7, **CHARACTERIZED IN THAT** the intermediate bracket forms a receiving space for the insertion of the holding element and at least a first loop end (12.1) of the penis loop (12) .

9. Excitation device (2) according to claim 6 to 8, **CHARACTERIZED IN THAT** the intermediate bracket (14) includes projections, which are insertable into different holes of a second loop end (12.2) of the penis loop (12), and, in this fixation, can be fixed for the target length.

10. Excitation device (2) according to claim 6 to 9, **CHARACTERIZED IN THAT** the intermediate bracket (14) comprises a bowl-shaped intake (14.1) with an circumferential edge to form a receiving space, onto which the clamp (4) as well as the two loop ends of the penis loop (6) are fixable, and that the intermediate bracket (14) comprises a pressure plate (14.2), that is can be inserted in the intake (14.1) in order to fix the penis loop (6) in the predetermined target length.

11. Excitation device (2) according to claim 10, **CHARACTERIZED IN THAT** the pressure plate (14.2) with a lower side presses on the first mounting end of the penis loop (6), the penis loop (6) then extends along a deflection region on the holding element and then extends back and can be fastened to the outside of the pressure plate (14.2).

12. Excitation device (2) according to claim 11, **CHARACTERIZED IN THAT** the pressure plate (14) comprises projections (14.2.1, 14.2.2) that intervene with holes on the penis loop (6) in order to adapt it to different penis sizes.

13. Excitation device (2) according to one of the preceding claims, **CHARACTERIZED IN THAT** this device includes a deflection region for the penis loop (12).

14. Excitation device (2) according to one of the preceding claims, **CHARACTERIZED IN THAT** on the outer side of the loop fastening means are provided for fasting on an intermediate member or the clamp (4) and that on the inner side of the loop at least one increase is provided to increase the contact pressure on the penis.

15. Excitation device (2) according to claim 1 to 14, **CHARACTERIZED IN THAT** the increase is designed as at least one pleasure rib (18.2) extending in the longitudinal direction of the loop and/or a pleasure nob (20.2).

## Revendications

1. Dispositif d'excitation (2) pour l'excitation intensive d'un organe sexuel masculin, de préférence un pénis, comprenant une unité de réception conçue pour entourer un organe sexuel masculin et un élément de retenue relié à celui-ci qui peut être mis en vibration au moyen d'un entraînement, d'un vibrateur ou similaire, l'unité de réception étant conçue comme une boucle de pénis élastique (6; 12; 18) fermée sur sa circonférence avec deux extrémités de boucle qui convergent ou sont reliées à l'élément de retenue, respectivement sont fixés à l'élément de retenue, dans lequel l'élément de retenue est conçu pour être fixé à un outil d'oscillation (10) et dans lequel l'élément de retenue est conçu comme un collier (4) comprenant une extrémité de fixation inférieure avec une ouverture de passage (4a) pour une fixation détachable à l'outil d'oscillation (10) et une extrémité de fixation supérieure pour une fixation aux extrémités de la boucle de pénis (6).

2. Dispositif d'excitation (2) selon la revendication 1, dans lequel le collier (4) comporte deux pattes (4b, 4c) espacées l'une de l'autre entre lesquelles les extrémités de fixation extrémités libres de la boucle de pénis peuvent être serrées.

3. Dispositif d'excitation (2) selon la revendication 1 ou 2, dans lequel le collier (4) est coudée entre une extrémité de fixation inférieure et l'extrémité de fixation supérieure dans la direction longitudinale, de préférence selon un angle d'environ 45 degrés.

4. Dispositif d'excitation (2) selon l'une des revendications précédentes, dans lequel la boucle de pénis (6) est réalisée au moins sur un côté intérieur de manière à créer un frottement.

5. Dispositif d'excitation (2) selon l'une des revendications précédentes, dans lequel la boucle de pénis (6) est réalisée de manière à être réglable en taille.

6. Dispositif d'excitation (2) selon l'une des revendications précédentes, ledit dispositif comprenant un support intermédiaire (14) conçu pour être relié à l'élément de retenue et à la boucle de pénis (12).

7. Dispositif d'excitation (2) selon la revendication 6, dans lequel le support intermédiaire (14) est conçu pour ajuster la circonférence de la boucle de pénis (12) sans outils.

8. Dispositif d'excitation (2) selon la revendication 6 ou 7, dans lequel le support intermédiaire forme un espace de réception pour l'insertion de l'élément de retenue et au moins une première extrémité de boucle (12.1) de la boucle de pénis (12).

9. Dispositif d'excitation selon les revendications 6 à 8, dans lequel le support intermédiaire (14) comprend des saillies qui peuvent être insérées dans différents trous d'une deuxième extrémité de boucle (12.2) de la boucle de pénis (12) et peuvent être fixées dans cette fixation pour la longueur souhaitée.

10. Dispositif d'excitation (2) selon les revendications 6 à 9, dans lequel le support intermédiaire (14) présente un réceptacle (14.1) en forme de cuvette avec un bord périphérique pour former un espace de réception auquel peuvent être fixées aussi bien le collier (4) que les deux extrémités de la boucle de pénis (6), et dans lequel le support intermédiaire (14) comprend une plaque de pression (14.2) qui peut être insérée dans le réceptacle (14.1) pour fixer la boucle de pénis (6) dans cette fixation pour la longueur prédéterminée souhaitée.

11. Dispositif d'excitation (2) selon la revendication 10, dans lequel la plaque de pression (14.2) appuie avec une face inférieure contre la première extrémité de fixation de la boucle de pénis (6), la boucle de pénis (6) passe ensuite à travers une déviation sur l'unité de retenue et revient ensuite et peut être fixée à l'extérieur sur la plaque de pression (14.2).

12. Dispositif d'excitation (2) selon la revendication 11, dans lequel des saillies (14.2.1, 14.2.2) sont prévues sur l'extérieur de la plaque de pression (14), qui s'engagent dans des trous sur la boucle de pénis (6) afin de l'adapter à différentes tailles de pénis.

13. Dispositif d'excitation (2) selon l'une des revendications précédentes, ledit dispositif d'excitation (2) comprenant une zone de déviation (12) pour la boucle de pénis.

14. Dispositif d'excitation (2) selon l'une des revendications précédentes, dans lequel des moyens de fixation pour la fixation à un support intermédiaire ou avec le collier (4) sont prévus sur l'extérieur de la boucle et dans lequel au moins une élévation pour augmenter la pression de contact sur le pénis est prévue sur le côté intérieur de la boucle.

15. Dispositif d'excitation (2) selon les revendications 1 à 14, dans lequel l'élévation est conçue comme au moins une nervure de luxure (18.2) s'étendant dans une direction longitudinale de la boucle et/ou un nœud de luxure (20.2).
